# DEMANDE DE BREVET EUROPEEN

(11) **EP 0 634 180 A1**
(43) Date de publication de la demande: **18.01.1995**
(21) Numéro de dépôt: 94420189.6
(22) Date de dépôt: 05.07.1994
(51) Int. Cl.: A61L 15/58

(54) **Bande médicale adhésive**

(30) Priorité: 13.07.1993 FR 9309063
(71) Demandeur: MOLINIER INDUSTRIES S.A., F-42340 Veauche (FR)
(72) Inventeur: Brunet, Sylvie, F-42110 Chambeon (FR); Favier, Roland, F-42340 Veauche (FR)
(74) Mandataire: Thivillier, Patrick

(57) **Abrégé**

La bande adhésive comprend un support textile (1) avec capacité d'élongation et dont l'une des faces au moins est soumise à une opération d'enduction d'une masse caoutchouteuse (2), tandis que la ou les faces ainsi traitées reçoivent une matière adhésive (3).

## Description

L'invention se rattache au secteur technique des bandages, plus particulièrement des bandes élastiques ou extensibles.

Il est apparu nécessaire, sur le plan médical et pour certaines applications thérapeutiques, que les bandages adhésifs, avec capacité de déformation élastique ou d'extensibilité, aient une rémanence importante. Or, les bandes actuellement mises sur le marché présentent certains inconvénients.

Selon une première forme de réalisation, la bande médicale est obtenue à partir d'un support textile incluant des fils de coton et recevant par enduction un adhésif. L'extensibilité de la bande est donnée par une surtension des fils de coton, ce qui nécessite un procédé de mise en oeuvre spécifique et très onéreux. Cette solution est donc d'un coût élevé.

On connait également, dans une autre forme de réalisation, des bandes médicales obtenues à partir d'un support textile quelconque, utilisant des fils élastiques ou des polyamides texturés afin de donner l'élasticité ou l'extensibilité recherchée au support. Une matière adhésive est appliquée sur l'une des faces au moins du support.

Cette solution est nettement moins onéreuse que la précédente mais ne permet pas d'obtenir une rémanence importante, compte-tenu de la contexture et de la mémoire spécifique de chaque fils de sorte que la bande revient toujours à sa position d'origine.

Il est apparu également nécessaire de pouvoir "casser la bande" avant de la poser sur le patient, c'est-à-dire qu'après avoir étiré la bande à son maximum pendant un temps déterminé, cette dernière ne revienne pas à sa position d'origine et ait perdu la quasi totalité de son extensibilité ou élasticité initiale.

L'invention s'est fixée pour but de remédier aux inconvénients des techniques connues, en ayant pour objectif d'obtenir une bande médicale extensible ou élastique et adhésive, d'un coût de revient adapté au prix du marché, et ayant une rémanence importante.

Pour résoudre ces problèmes, il a été conçu et mis au point une bande médicale adhésive qui comprend un support textile avec capacité d'élongation et dont l'une des faces au moins est soumise à une opération d'enduction d'une masse caoutchouteuse, tandis que la ou les faces ainsi traitées recoivent une matière adhésive.

Le support est obtenu à partir d'un tissu tissé tricoté ou non tissé, avec ou sans lisières, en ayant par exemple en chaîne des fils élastiques, la structure obtenue pouvant être légère et aérée.

Pour résoudre le problème posé de tenir compte de l'élasticité du support textile, l'enduction de la masse caoutchouteuse s'effectue par projection d'une émulsion aqueuse de gomme naturelle ou synthétique, successivement sur l'une puis l'autre face de la bande ou sur une seule face de cette dernière. Notamment, cette opération d'enduction s'effectue comme indiqué dans la demande de brevet FR 8703355 dont le demandeur est également titulaire. Cette technique garantit la qualité régulière tout au long du traitement.

Avantageusement, I'émulsion aqueuse de gomme naturelle ou synthétique est du latex.

La matière adhésive est appliquée sous forme de bandes parallèles entre elles et aux lisières ou sur la totalité ou une partie de la surface de la bande, sans pour cela exclure d'autres procédés.

L'invention est exposée, ci-après plus en détail à l'aide des dessins annexés, dans lesquels :

Les figures 1 et 2 sont des vues en plan du support textile de base respectivement avant et après élongation.

La figure 3 est une vue en coupe à caractère purement schématique, montrant l'article textile avant enduction.

La figure 4 est une vue correspondant à la figure 3 montrant l'enduction de latex selon les deux faces de l'article textile.

La figure 5 est à une échelle plus importante, une vue en coupe de l'article montrant l'application d'une matière adhésive sur une face enduite de l'émulsion aqueuse de gomme.

La figure 6 est une vue de dessous correspondant à la figure 5.

La figure 7 est une vue en coupe à caractère schématique montrant l'article équipé d'un film protecteur.

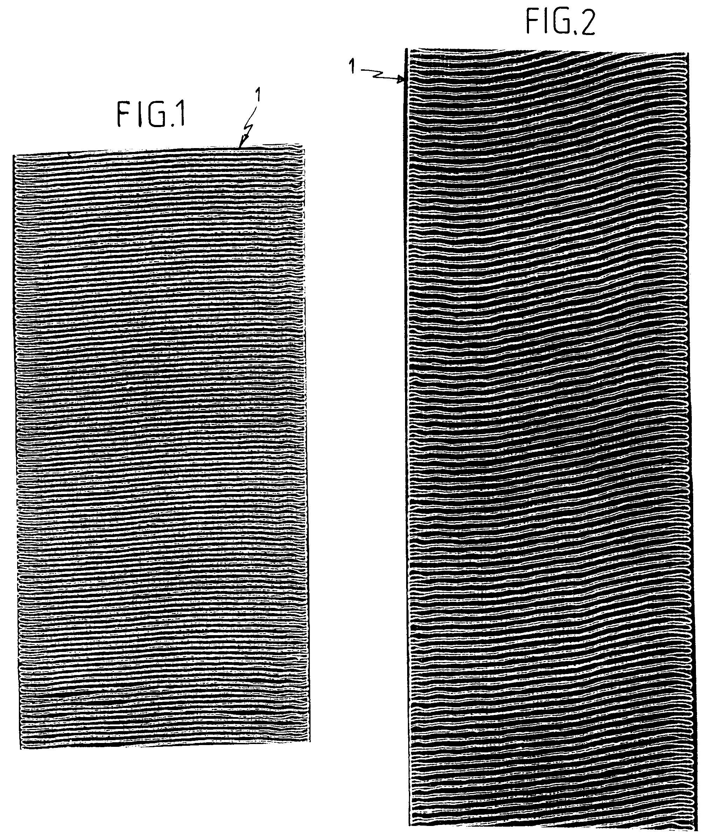

La bande médicale, selon l'invention, est obtenue à partir d'un support textile (1) tricoté, tissé ou non tissé, avec ou sans lisières. Le support (1) présente longitudinalement par exemple, des fils de chaîne élastiques entrecroisés avec des fils de trame non élastiques. Ce support est conformé avantageusement pour constituer une structure légère et aérée sans pour cela exclure un autre aspect du support. La figure 1 montre le support en position non étirée, tandis que la figure 2 le montre en position d'allongement.

Ce support, ainsi défini dans sa structure de base, est soumis sur l'une de ses faces au moins, à une opération d'enduction d'une matière caoutchouteuse (2). Notamment, l'une des faces du support (1) et, de préférence les deux, est soumise à une projection d'émulsion aqueuse de gomme naturelle, du type latex liquide, sans pour cela exclure d'autres masses liquides adhésives de caoutchouc, synthétique ou naturelle.

Avantageusement, cette opération d'enduction s'effectue dans les conditions définies dans la demande de brevet FR 8703355 dont le demandeur de la présente est également titulaire.

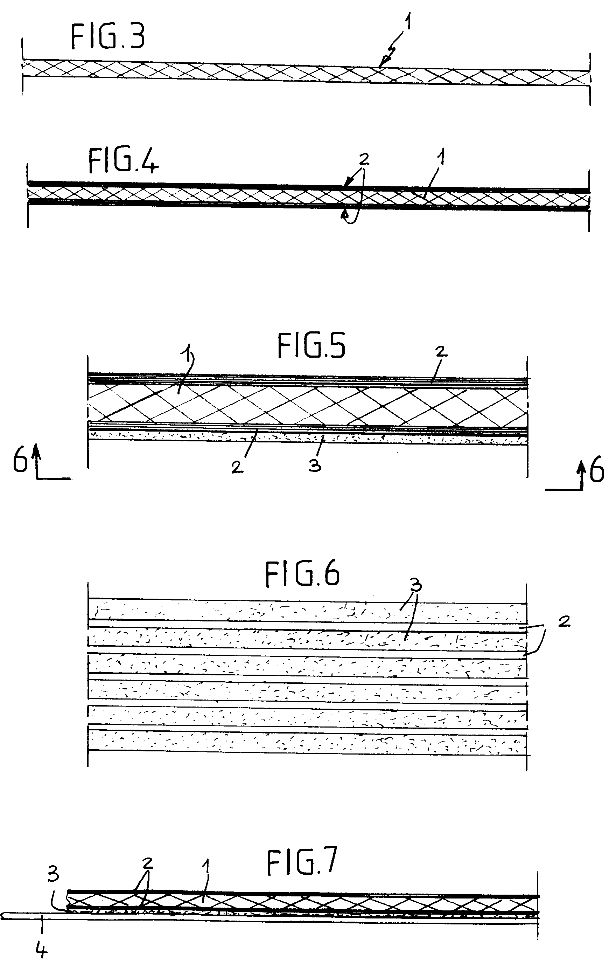

Cette opération d'enduction de gomme caoutchouteuse liquide, confère à la bande, une force de contention importante, permettant d'obtenir une pression de contention indispensable dans le cas d'un bandage circulaire compressif. La figure 4 montre le support textile (1) dont les deux faces ont été soumise à une opération d'enduction au moyen d'une gomme naturelle du type latex (2). A ce stade, la bande obtenue présente des caractéristiques de cohésivité.

L'une des faces au moins ainsi traitée du support (1), c'est-à-dire soumise à la projection du latex (2), est traitée pour être rendue adhésive. Cette opération d'adhésivage s'effectue par tout moyen connu et approprié. Par exemple, la matière adhésive (3) est appliquée sous forme de bandes longitudinales parallèles entre elles et aux lisières (figures 5 et 6). A titre indicatif, le film de colle (3) peut être appliqué sur la face correspondante du support préalablement enduite de latex (2), par léchage au fur et à mesure du défilement dudit support. Par exemple, l'application de l'adhésif peut être effectuée comme enseigné par le brevet FR 8804478 dont le demandeur de la présente est également titulaire.

Il apparait donc, que le fait de soumettre une bande extensible ou élastique à une opération d'enduction de latex, faisant office d'apprêt à une matière adhésive, permet d'obtenir un bandage adhésif ayant une rémanence importante en ayant, de plus, la caractéristique de ne pas revenir à sa position d'origine après un étirement maximum pendant un temps déterminé, la bande ayant perdu la quasi totalité de son élasticité ou extensibilité initiale. La combinaison des opérations de cohésivage et d'adhésivage permet d'avoir une tenue exceptionnelle de la bande après sa pose.

L'enduction de latex facilite l'accrochage d'une matière adhésive ou tout autre substance, quel que soit le type de support (1) permettant notamment, d'utiliser des supports très aérés et très légers, d'application aisée, tout en respectant les fonctions cutanées, compte-tenu d'une bonne perméabilité à l'air et de son hypoallergénicité.

Bien évidemment, la bande médicale ainsi traitée selon les caractéristiques de l'invention, peut être revêtue du côté de sa face rendue adhésive, d'un film protecteur (4) mais peut également être roulée sans film protecteur.

Les avantages ressortent bien de la description, en particulier on souligne :
- la possibilité de réaliser un support très léger et aéré,
- la simplicité de mise en oeuvre,
- la possibilité d'obtenir une force de contention adaptée en fonction de la masse caoutchouteuse,
- la possibilité de conditionner la bande sans film protecteur.

## Revendications

**-1-** Bande médicale adhésive élastique ou extensible caractérisée en ce qu'elle comprend un support textile (1) avec capacité d'élongation et dont l'une des faces au moins est soumise à une opération d'enduction d'une masse caoutchouteuse (2), tandis que la ou les faces ainsi traitées recoivent une matière adhésive (3).

**-2-** Bande médicale selon la revendication 1, caractérisée en ce que l'enduction de la masse caoutchouteuse (2) s'effectue par projection d'une émulsion aqueuse de gomme naturelle ou synthétique, successivement sur l'une puis l'autre face du support.

**-3-** Bande médicale selon la revendication 1, caractérisée en ce que l'enduction de la masse caoutchouteuse (2) s'effectue par vaporisation sur l'une des faces du support.

**-4-** Bande médicale selon la revendication 2, caractérisée en ce que l'émulsion aqueuse de gomme naturelle (2) est du latex.

**-5-** Bande médicale selon la revendication 1, caractérisée en ce que le support (1) est obtenu à partir d'un tissu tricoté, tissé ou non tissé, avec ou sans lisières, en étant conformé pour obtenir une structure légère et aérée.
